(19) Europäisches Patentamt
European Patent Office
Office européen des brevets

(11) Numéro de publication : 0 280 593 B1

# (12) FASCICULE DE BREVET EUROPEEN

(45) Date de publication du fascicule du brevet : **12.06.91 Bulletin 91/24**

(21) Numéro de dépôt : **88400156.1**

(22) Date de dépôt : **25.01.88**

(51) Int. Cl.$^5$ : **A61K 31/195**, A61K 31/70, A61K 31/51, A61K 31/595, // (A61K31/70, 31:195, 31:185, 31:355, 31:595, 31:525, 31:51, 31:455, 31:44, 31:375)

## (54) Composition à base d'acide aminé et de vitamines utilisable en thérapeutique cancérologique.

(30) Priorité : **26.01.87 FR 8700845**

(43) Date de publication de la demande : **31.08.88 Bulletin 88/35**

(45) Mention de la délivrance du brevet : **12.06.91 Bulletin 91/24**

(84) Etats contractants désignés : **BE CH DE ES FR GB IT LI LU NL SE**

(56) Documents cités :
**UNLISTED DRUGS, vol. 23, no. 5, mai 1971, page 67a, Chatham, New Jersey, US; "Llanol"**
**UNLISTED DRUGS, vol. 27, no. 9, septembre 1975, page 143k, Chatham, New Jersey, US; "Estaron S"**

(56) Documents cités :
**UNLISTED DRUGS, vol. 28, no. 7, juillet 1976, page 117p, Chatham, New Jersey, US; "Hizeneck-D"**
**UNLISTED DRUGS, vol. 35, no. 7, juillet 1983, page 115f, Chatham, New Jersey, US; "Phakolen"**
**"Dictionnaire Vidal", 1972, page 1179, O.V.P., PARIS, FR; "Phakan"**

(73) Titulaire : **Rougereau, André**
**23, rue Jules Simon**
**F-37000 Tours (FR)**

(72) Inventeur : **Rougereau, André**
**23, rue Jules Simon**
**F-37000 Tours (FR)**

(74) Mandataire : **Battut, Michel**
**OFFICE PICARD 134, boulevard de Clichy**
**F-75018 Paris (FR)**

EP 0 280 593 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention concerne une nouvelle composition utilisable en thérapeutique, et plus particulièrement une association à base d'acide aminé, d'une part, et de vitamines, d'autre part, utilisable en thérapeutique cancérologique.

Divers traitements thérapeutiques du cancer ont été proposés en chirurgie, en radiothérapie et en chimiothérapie.

Ainsi, les exérèses chirurgicales, dont les indications sont tributaires de la localisation et de l'envahissement de la tumeur, peuvent être satisfaisantes sur le plan carcinologique, ou simplement palliatives. Cependant, elles constituent une agression certaine pour l'organisme et peuvent entraîner des mutilations définitives.

La radiothérapie, isolée ou associée à un autre traitement, possède une action indéniable sur certains types de tumeurs, mais son action dépasse toujours le cadre régional de la tumeur, entraînant des séquelles qui dépendent de la localisation. Le retentissement sur l'état général du patient dépend des doses cumulées rendues nécessaires en fonction de la masse traitée.

En chimiothérapie, on utilise par exemple des sels de métaux lourds, des agents d'alkylation tels que le cyclophosphamide ou la méchlorétamine, des antimétabolites tels que des dérivés d'uracile ou de cytosine, certains dérivés de la série des antibiotiques comme la bléomycine ou la daunomycine, ou encore des alcaloïdes tels que la vinblastine et la vincristine. Ces composés peuvent être utilisés, selon les cas, isolément ou en association de deux ou plusieurs. Ils présentent généralement des effets secondaires toxiques qui limitent ou interdisent leur utilisation à doses élevées, et ce d'autant plus si le patient présente des tares associées ou des séquelles de chirurgie mutilante ou de radiothérapie.

Toutes ces thérapeutiques, à des degrés divers, ont pour but d'éliminer le maximum ou la totalité des tumeurs cancéreuses, mais aucune d'elles n'exerce d'action sur le génie évolutif du processus cancéreux.

On connait l'importance des vitamines, hydrosolubles et liposolubles, en physiologie et biochimie nutritionnelles. Par contre, leur utilisation pour le traitement du cancer, isolément ou en combinaison avec d'autres composés, n'a pas été décrite jusqu'à présent.

D'autre part, certains acides aminés sont utilisés en thérapeutique pour le traitement de diverses maladies. Ainsi par exemple on sait que la β-alanine possède des propriétés inhibitrices de la vasodilatation périphérique permettant son administration pour le traitement des bouffées de chaleur de la ménopause. Sa toxicité est faible puisque la dose léthale $DL_{50}$ par voie orale chez la souris et le rat, est supérieure à 10 g/kg, et sa tolérance est généralement satisfaisante. Si l'activité antiallergique et antihistaminique de la β-alanine a été décrite, on ne connait par contre aucune application de ce composé, isolément ou en combinaison, au traitement du cancer.

Il est également admis que la β-alanine agit comme médiateur chimique au niveau du système nerveux central, et pourrait agir au niveau cellulaire par rapport au signal calcium.

On connait quelques compositions contenant divers composants, parmi lesquels certains acides aminés et des vitamines ; par exemple "Unlisted Drug" vol. 28 n° 7 cite une composition utilisable comme supplément nutritionnel, contenant de la pyridoxine, de l'acide citrique, de la taurine, etc. Toutefois, l'utilisation spécifique de la β-alanine associée à des vitamines n'a pas été envisagée.

On a maintenant constaté qu'une composition contenant de la β-alanine en association avec diverses vitamines, présente des propriétés permettant son application en thérapeutique pour le traitement du cancer, tandis qu'aucune activité comparable n'est observée quand on utilise isolément chacun de ces composés.

La présente invention a donc pour objet une nouvelle composition à base de β-alanine et de vitamines, utilisable pour le traitement du cancer.

La nouvelle composition conforme à la présente invention comprend au moins une vitamine et de la β-alanine représentée par la formule générale (I) suivante :

$$H_2N\text{-}(CH_2)_2\text{-}COOH \quad (I)$$

ou un de ses sels d'acide ou de base.

Plus particulièrement, la composition conforme à l'invention comprend la β-alanine répondant à la formule (I) ci-dessus et une ou plusieurs vitamines choisies parmi la vitamine A, la vitamine $B_1$, la vitamine $B_2$, la vitamine $B_5$, la pyridoxine, l'acide ascorbique, le tocophérol, la vitamine $D_2$, et la vitamine PP.

La β-alanine peut être utilisée isolément ou, le cas échéant, en combinaison avec un ou plusieurs autres acides aminés ; par exemple la β-alanine et la glycine, ou la β-alanine et la taurine, peuvent être combinées.

Outre les vitamines et l'acide aminé indiqués ci-dessus, la composition peut également avantageusement comporter des additifs tels que le glucose, le β-carotène, ou d'autres vitamines telles que la biotine et la vitamine $B_{12}$.

Suivant une forme préférentielle de réalisation de l'invention, la composition comprend la β-alanine, et plusieurs vitamines choisies parmi celles indiquées ci-dessus, ainsi que du glucose, et éventuellement de la taurine, et/ou de la glycine. Il peut être préférable d'ajouter à l'ensemble de ces constituants du β-carotène, précurseur de la vitamine A.

Le rapport pondéral de l'acide aminé au total des vitamines est compris entre 50/1 et 500/1, et de préférence entre 100/1 et 300/1, et la quantité d'acide aminé administrée par jour est comprise entre 50 g et 200 g pour un traitement d'attaque, et entre 10 et 50 g pour un traitement d'entretien chez l'homme adulte. Bien entendu, la dose administrée est déterminée par le médecin en fonction de l'état du patient. Lorsque du glucose est utilisé, il peut représenter entre 10 et 30% en poids de la dose d'acide aminé pour un traitement d'attaque. La quantité de glucose utilisée est sensiblement la même dans un traitement d'attaque et dans un traitement d'entretien.

L'invention concerne également un procédé pour préparer une composition utile en thérapeutique cancérologique.

Les composants constituant la composition de la présente invention peuvent être administrés en mélange ou séparément, de préférence par voie orale. On peut par exemple mélanger l'acide aminé, les vitamines et, le cas échéant, le glucose et les autres additifs, sous forme d'une poudre que l'on peut conditionner sous diverses formes usuelles, par exemple en sachets, en gélules ou capsules. Il est également possible de conditionner séparément l'acide aminé, d'une part, et les vitamines et les autres additifs éventuels, d'autre part, par exemple sous forme de gélules ou capsules administrables par voie orale, séparément, l'administration de chacune de ces deux parties pouvant être espacée dans le temps de quelques minutes à quelques heures, sans excéder toutefois 4 à 5 heures environ.

La composition conforme à la présente invention peut constituer une thérapeutique d'accompagnement des traitements thérapeutiques usuels, chimiques, chimiothérapiques ou radiothérapiques, dans le cas du traitement du cancer. L'avantage de la thérapeutique conforme à la présente invention réside notamment dans le fait que les taitement connus, qui, comme indiqué précédemment, sont traumatisants, voire toxiques, peuvent désormais être utilisés à des doses plus faibles qui évitent les effets secondaires néfastes.

Les résultats expérimentaux ont montré que la composition suivant l'invention ne détruit pas la cellule cancéreuse, mais inhibe sa division cellulaire. Elle possède la propriété, comme les antimitotiques et la radiothérapie, d'agir sur la carcinomédine, potentialisateur de la multiplication cellulaire tumorale. Il est particulièrement intéressant de noter que ces résultats sont obtenus quand les composants sont administrés simultanément, en mélange, mais aussi lorsque les vitamines, d'une part, et l'acide aminé, d'autre part, sont administrés séparément à un intervalle de temps allant de quelques minutes à 4 à 5 heures environ.

Par contre, aucun effet de destruction sur les cellules cancéreuses n'est observé si on administre uniquement un acide aminé, ou des vitamines, ou si leur rapport pondéral ou les doses administrées sont en dehors des limites précitées.

Les études toxicologiques ont montré que la toxicité de la composition suivant l'invention est nulle aux doses administrées pour le traitement du cancer.

Des exemples de compositions conformes à la présente invention sont donnés ci-après à titre non limitatif.

EXEMPLE 1

On prépare un mélange des composants ci-indiqués ci-dessous :

| | | |
|---|---|---|
| β-alanine | 90 | g |
| taurine | 10 | g |
| Glucose | 30 | g |
| Vitamine A | 60.000 | UI |
| Vitamine $B_1$ | 24 | mg |
| Vitamine $B_2$ | 18 | mg |
| Vitamine $B_5$ | 48 | mg |
| Pyridoxine (chlorhydrate) | 24 | mg |
| Acide ascorbique | 600 | mg |
| Vitamine $D_2$ | 12.000 | UI |
| Tocophérol (acétate) | 24 | mg |
| Vitamine PP | 120 | mg |
| β-carotène | 100 | mg |

Ces composants sont soigneusement mélangés de manière à obtenir une composition homogène correspondant à la dose administrée en 24 heures pour un traitement d'attaque. L'administration est faite par voie orale en quatre parties égales, toutes les 6 heures, soit par prise volontaire, soit additionnée à la solution de nutrition entérale.

EXEMPLE 2

On procède comme dans l'exemple 1 en utilisant les composants ci-dessous pour préparer une composition destinée à un traitement d'entretien :

| | | |
|---|---|---|
| β-alanine | 30 | g |
| Glucose | 10 | g |
| Vitamine A | 15.000 | UI |
| Vitamine $B_1$ | 7,5 | mg |
| Vitamine $B_2$ | 7,5 | mg |
| Vitamine $B_5$ | 6,5 | mg |
| Pyridoxine (chlorhydrate) | 2,3 | mg |
| Biotine | 0,1 | mg |
| Vitamine $B_{12}$ | 4,5 | mg |
| Acide ascorbique | 112 | mg |
| Vitamine $D_2$ | 3.000 | UI |
| Tocophérol (acétate) | 7,5 | mg |
| Vitamine PP | 37,5 | mg |
| β-carotène | 60 | mg |

On obtient ainsi une quantité de composition correspondant à la dose administrée en 24 heures au cours

d'un traitement d'entretien. La composition ci-dessus est administrée en quatre parties égales, toutes les 6 heures.

EXEMPLE 3

En procédant comme dans l'exemple 1, on prépare un mélange des composants suivants :

| | | |
|---|---|---|
| β-alanine | 20 | g |
| Vitamine $B_1$ → see below | | |

Le mélange est homogénéisé puis réparti entre 5 sachets identiques pour administration par voie orale. Par ailleurs, on prépare, de manière usuelle, 5 sachets dosés à 4 g de taurine.

La composition est administrée à raison de cinq prises d'un sachet du mélange de vitamines et de β-alanine ci-dessus, espacées de quatre heures, suivies à une heure d'intervalle par cinq prises d'un sachet de taurine. On a ainsi un traitement d'attaque qui est renouvelé pendant une durée qui dépend de l'état clinique du patient.

## Revendications

1. Composition pour utilisation comme médicament, notamment utilisable dans le traitement du cancer, caractérisée en ce qu'elle comprend au moins une vitamine et de la β-alanine représentée par la formule générale (I) suivante :

$$H_2N\text{-}(CH_2)_2\text{-}COOH$$

ou un de ses sels d'acide ou de base.

2. Composition selon la revendication 1, caractérisée en ce qu'elle comprend une ou plusieurs vitamines choisies parmi la vitamine A, la vitamine $B_1$, la vitamine $B_2$, la vitamine $B_5$, la pyridoxine, l'acide ascorbique, le tocophérol, la vitamine $D_2$, et la vitamine PP.

3. Composition selon la revendication 1, caractérisée en ce qu'elle contient en outre de la taurine et/ou de la glycine.

4. Composition selon l'une quelconque des revendications 1 à 3, caractérisée en ce qu'elle comprend en outre au moins un additif choisi parmi le glucose, le β-carotène, la biotine et la vitamine $B_{12}$.

5. Composition selon l'une quelconque des revendications 1 et 2, caractérisée en ce qu'elle comprend la β-alanine, la taurine et/ou la glycine, au moins une vitamine, et du glucose.

6. Composition selon la revendication 1, caractérisée en ce que le rapport pondéral de l'acide aminé au total des vitamines est compris entre 50/1 et 500/1.

7. Composition selon l'une quelconque des revendications 1 à 6, caractérisée en ce qu'elle est sous forme d'un mélange de ses composants, administrable par voie orale.

8. Procédé de préparation d'une composition pour utilisation comme médicament, notamment dans le traitement du cancer selon l'une quelconque des revendications 1 à 6, caractérisé en ce qu'on mélange la β-alanine, les vitamines, et les autres composants, le cas échéant, sous forme de poudre.

## Ansprüche

1. Zusammensetzung für die Verwendung als Arzneimittel, das insbesondere für die Behandlung von Krebs verwendbar ist, dadurch gekennzeichnet, daß sie umfaßt mindestens ein Vitamin und β-Alanin der nachstehend

angegebenen allgemeinen Formel (I) :

$$H_2N\text{-}(CH_2)_2\text{-}COOH$$

oder eines seiner Säure- oder Basensalze.

2. Zusammensetzung nach Anspruch 1, dadurch gekennzeichnet, daß sie umfaßt ein oder mehrere Vitamine, ausgewählt aus der Gruppe Vitamin A, Vitamin $B_1$, Vitamin $B_2$, Vitamin $B_5$, Pyridoxin, Ascorbinsäure, Tocopherol, Vitamin $D_2$ und Vitamin PP.

3. Zusammensetzung nach Anspruch 1, dadurch gekennzeichnet, daß sie außerdem Taurin und/oder Glycin enthält.

4. Zusammensetzung nach einem der Ansprüche 1 bis 3, dadurch gekennzeichnet, daß sie außerdem mindestens ein Additiv, ausgewählt aus der Gruppe Glucose, β-Karotin, Biotin und Vitamin $B_{12}$, enthält.

5. Zusammensetzung nach einem der Ansprüche 1 und 2, dadurch gekennzeichnet, daß sie umfaßt β-Alanin, Taurin und/oder Glycin, mindestens ein Vitamin und Glucose.

6. Zusammensetzung nach Anspruch 1, dadurch gekennzeichnet, daß das Gewichtsverhältnis zwischen der Aminosäure und der Gesamtmenge der Vitamine zwischen 50 : 1 und 500 : 1 liegt.

7. Zusammensetzung nach einem der Ansprüche 1 bis 6, dadurch gekennzeichnet, daß sie in Form einer auf oralem Wege verabreichbaren Mischung ihrer Bestandteile vorliegt.

8. Verfahren zur Herstellung einer Zusammensetzung für die Verwendung als Arzneimittel, insbesondere für die Behandlung von Krebs, nach einem der Ansprüche 1 bis 6, dadurch gekennzeichnet, daß man das β-Alanin, die Vitamine und gegebenenfalls die anderen Bestandteile. in Form eines Pulvers miteinander mischt.

**Claims**

1. A composition to be used as a drug, namely for use in the cancer therapy, characterized in that it comprises at least one vitamin and β-alanine, represented by the following general formula (I) :

$$H_2N\text{-}(CH_2)_2\text{-}COOH$$

or one of its base or acid salts

2. A composition according to claim 1, characterized in that it comprises one or several vitamins selected from the group consisting of vitamin A, vitamin $B_1$, vitamin $B_2$, vitamin $B_5$, pyridoxine, ascorbic acid, tocopherol, vitamin $D_2$ and vitamin PP.

3. A composition according to claim 1, characterized in that, it further contains taurine and/or glycine.

4. A composition according to any of claims 1 to 3, characterized in that it further comprises at least one additive selected from the group consisting of glucose, β-carotene, biotin and vitamin $B_{12}$.

5. A composition according to any of claims 1 and 2, characterized in that it comprises β-alanine, taurine and/or glycine, at least one vitamin and glucose.

6. A composition according to claim 1, characterized in that the weight ratio of the aminoacid to the total of vitamins is comprised between 50/1 and 500/.1.

7. A composition according to any of claims 1 to 6, characterized in that it is in the form of a mixture of its components, orally dispensable.

8. A process for preparing a composition to be used as a drug, namely for use in the cancer therapy according to any of claims 1 to 6, characterized in that β-alanine, vitamins and other components, optionally, are mixed together in the form of a powder.